# EUROPEAN PATENT SPECIFICATION

(11) **EP 2 621 584 B1**
(45) Date of publication and mention of the grant of the patent: **14.01.2015**
(21) Application number: 11770258.9
(22) Date of filing: 28.09.2011
(51) Int. Cl.: A61N 1/32

(54) **NANOWIRES FOR ELECTROPHYSIOLOGICAL APPLICATIONS**
NANOWIRES FÜR ELEKTROPHYSIOLOGISCHE ANWENDUNGEN
NANOWIRES DESTINÉS À DES APPLICATIONS ÉLECTROPHYSIOLOGIQUES

(30) Priority: 14.03.2011 US 201161452283 P; 29.09.2010 US 387604 P
(43) Date of publication of application: 07.08.2013
(73) Proprietor: President and Fellows of Harvard College, Cambridge, MA 02138 (US)
(72) Inventor: PARK, Hongkun, Cambridge Massachusetts 02138 (US); ROBINSON, Jacob, Cambridge Massachusetts 02138 (US); SUTTON, Amy, Cambridge Massachusetts 02138 (US); JORGOLLI, Marsela, Cambridge Massachusetts 02138 (US); SHALEK, Alexander Kann, Cambridge Massachusetts 02138 (US)
(74) Representative: Hoffmann Eitle
(86) International application number: PCT/US2011/053640
(87) International publication number: WO 2012/050876

(56) References cited:
- WO-A1-2004/036202
- WO-A1-2005/093831
- WO-A1-2009/104056
- US-A1- 2007 187 840

## Description

### BACKGROUND

Cellular electrophysiology is the primary means for discovering and characterizing ion channels and their associated agonists and blockers. It is also used to investigate electrical and chemical characteristics of individual cells and multi-cellular interactions.

Traditionally, electrophysiological measurements are performed by using individual manually-operated patch pipettes, which are limited in scale and throughput. Recently, a variety of approaches for parallel electrophysiology measurements have led to improved throughput. Examples include planar patch clamp techniques, multielectrode arrays, and vertically aligned carbon nanotubes or fibers. See, e.g., Brüggemann, A. et al., Methods in Molecular Biology 491, 165-176 (2008); Pine, J., Advances in Network Electrophysiology, 3-23 (2006); Voelker, M. & Fromherz, P., Small 1, 206-210 (2005); Patolsky, F. et al., Science 313, 1100-1104 (2006); and Yu, Z. et al., Nano Letters 7, 2188-2195 (2007). Yet, these techniques cannot be widely adopted because of their intrinsic limitations such as high fabrication costs.

There is a demand for a new method capable of conferring a high-throughput low-cost cellular electrophysiological measurement.

US 2007/0187840 discloses a device comprising a planar integrated circuit that includes an array of electrodes and at least one nanostructure in electrical contact with at least one electrode. WO 2004/036 202 relates to methods of measuring electrical properties of a cell using nanoelectrodes for invention into a cell.

### SUMMARY

This invention relates to a device containing electrically conductive nanowires and its application in parallel high-throughput cellular electrophysiological measurements. The invention is set out in the appended claims.

In one aspect, this invention features an electrical device including a substrate having a surface, which is coated with an electrically insulted layer, and a plurality of electrically conductive nanowires, each of which, having a first end and a second end, is coated with an electrically insulating layer except for the first and second ends, the first end being attached to the surface and the second end being coated with an electrically conductive layer. Each NW can be individually addressable by a voltage waveform.

The electrically conductive nanowires used in the above-described device can be made of a semiconductor (e.g., Si and Ge), a compound semiconductor, a metal oxide (e.g., ZnO), a metal (e.g., Au, Ag, Ir, Pt), carbon, boron nitride, or a combination thereof.

The term "compound semiconductor" refers to a semiconductive compound formed of two or more elements such as IV-IV semiconductors (e.g., SiC and SiGe), III-V semiconductors (e.g., AlN, AlP, AlGaAs, GaN, GaAs, InP, and InGaAs), II-V semiconductors (e.g., Zn₃Sb₂ and Cd₃As₂), II-VI semiconductors (e.g., CdS, CdSe, CdTe, IV-VI semiconductors (e.g., SnS and PbSnTe), I-VI semiconductors (e.g., Cu₂S), I-VII semiconductors (e.g., CuCl), and oxide semiconductors (e.g., SnO₂, CuO, and Cu₂O). Unless stated otherwise, the semiconductor used for the electrical device of this invention includes both its intrinsic form (i.e., pure form) and doped form (i.e., containing one or more dopants). The term "combination" refers to a mixture, an alloy, or a suitable reaction product of two or more components. For example, the term "a combination of silicon and a metal" refers to both a mixture of silicon and the metal and a silicide of the metal.

The term "nanowire" (or "NW") refers to a material in the shape of a wire or rod having a diameter in the range of 1 nm to 1 µm. The NWs are attached to the surface along a substantially vertical direction (i.e., 60-90 degree) to the surface. They each can have a diameter of 10 nm-500 nm (e.g., 50-250 nm or 90-180 nm), and a length of 20 nm-10 µm (e.g., 50 nm-5 µm). The device has a nanowire density, i.e., wires per unit area of 0.05-10 wires µm⁻² (e.g., 0.5-5 wires µm⁻² or 1-2 wires µm⁻²).

The electrically insulating layer is formed of an inorganic material such as an oxide (e.g., silica, alumina, and hafnium oxide) and a nitride (e.g., silicon nitride). Alternatively, the electrically insulating layer is formed of an organic material such as Parylene (e.g., Parylene C, N, AF-4, SF, HT, A, AM, VT-4, or CF) and polydimethylsiloxane, methyl methacrylate, a photoresist (e.g., SU-8) and an electron beam resist (e.g., polymethylmethacrylate, ZEP-520, and hydrogen silsesquioxane).

The electrically conductive layer is formed of a semiconductor (e.g., Si and Ge), a compound semiconductor, a metal (e.g., Ag, Au, Pt, Ni, Al, Pd, W, Ti, and Cr), a metal oxide (e.g., indium tin oxide), a metal nitride (e.g., titanium nitride), or a combination thereof (e.g., a metal silicide).

The electrically conductive layer can include a metal top layer and a metal silicide intermediate layer between the metal top layer and the second end. The metal silicide can be a silicide of Pt, Ni, W, Pd, Ti, Cr, Yb, Er, Tb, Dy, Gd, Ho, Y, Hf, Zr, Ta, Co, V, Mo, Rh, Ir, or a combination thereof. Examples of silicide include but are not limited to PtSi, Pt₂Si, NiSi, Ni₂Si, NiSi₂, WSi₂, Pd₂Si, TiSi₂, CrSi₂, YbSi₂, ErSi₂, TbSi₂, DySi₂, GdSi₂, HoSi₂, YSi₂, HfSi, ZrSi₂, TaSi₂, CoSi₂, VSi₂, CoSi, MoSi₂, RhSi, Ir₂Si₃, IrSi, and IrSi₃.

The plurality of electrically conductive nanowires in the device described above can include a first plurality of electrically conductive nanowires and a second plurality of electrically conductive nanowires. The first plurality of nanowires can be in electrical communication with each other. They can each be electrically insulated from the second plurality of electrically conductive nanowires. The first and second pluralities of NWs may be the same or different in terms of composition and configuration. Alternatively, at least two of the plurality of nanowires can be electrically insulated from each other.

The substrate can be formed of a semiconductor (e.g., Si), a compound semiconductor (e.g., GaAs, InP, GaN, and GaP), or diamond.

In another aspect, this invention relates to a in vitro method of sending or receiving an electrical signal to or from a biological cell (such as a stem cell, an immune cell, and a primary cell cultured on a surface. The method includes providing the device described above and contacting the biological cell with the device to allow penetration of at least one nanowire into the cell, whereby the second end of the at least one nanowire is located inside the cell for sending or receiving the electrical signal.

This method may include one or more of the following features. The biological cell can be a neuron, a neuroblast, an HEK cell, a HeLa cell, an oocyte, a beta cell, a myocyte, an osteocyte, a fibroblast, a macrophage, or a stem cell. The method may further include contacting a second biological cell with the device to allow penetration of a second nanowire that is electrically insulated from the at least one nanowire, whereby the second end of the second nanowire is located inside the second biological cell for sending and receiving a second electrical signal. The biological cell and the second biological cells can both be neurons. Preferably, each biological cell is penetrated by two or more NWs in electrical communication with each other. The electrical signal can either be an electrical current or voltage signal. For example, characterization of ion channels can be achieved by measuring the voltage and current responses of a live cell via the current and voltage clamp experiments respectively.

The device described above can be used for electrophysiological measurement both *in vitro*. When conducting *in vitro* measurements, cells can be cultured directly on the device or cultured on another substrate which is then placed atop the device with the cell-side facing it. Alternatively, in another aspect which does not form part of the present invention, the device can be implanted *in vivo.* To facilitate integration, the device may contain a layer of cultured cells.

One advantage of the invention is a high-throughput device production. More specifically, with conventional semiconductor fabrication technologies, a large amount of arrays of NWs can be quickly produced on semiconductor wafers to obtain the devices described above. This high-throughput fabrication, combined with the reusability of these devices, enables the low-cost production and implementation of the device of this invention. Other advantages include that NWs penetrating the cellular membrane as intracellular electrodes do not compromise cell viability; that the device has a high sensitivity to electrical signals (e.g., a voltage change of less than 10 mV in membrane potential); and that the device can easily interface with standard optical stimulation and recording techniques (including optogenetic methods and use of voltage or calcium sensitive dyes).

The details of one or more embodiments are set forth in the accompanying description below. Other aspects, features, and advantages will be apparent from the following drawing, detailed description of embodiments, and also from the appending claims. The aspects, examples and embodiments of the present description which do not fall within the scope of the appended claims are provided for illustrative purposes only and do not form part of the invention as set out in the appended claims.

### BRIEF DESCRIPTION OF DRAWINGS

Figures 1(a) and 1(b) are schematics of a device of the present invention for electrophysiological applications.
Figure 2 illustrates a process of fabricating a device of this invention, the process including steps (a) through (i).
Figure 3(a) is an optical micrograph of a device of this invention and Figure 3(b) is a picture of a device having PDMS wells disposed thereon for containing a cell culture bath. Scale bars: (a) 200 µm, (b) 1 cm.
Figures 4(a) and 4(b) are scanning electron micrographs (SEMs) of a rat hippocampal neuron cultured on the device of Figure 3(b). Scale bars in both Figures 4(a) and 4(b): 5 µm.
Figure 5 illustrates use of the device of Figure 3(b) for electrophysiological measurements.

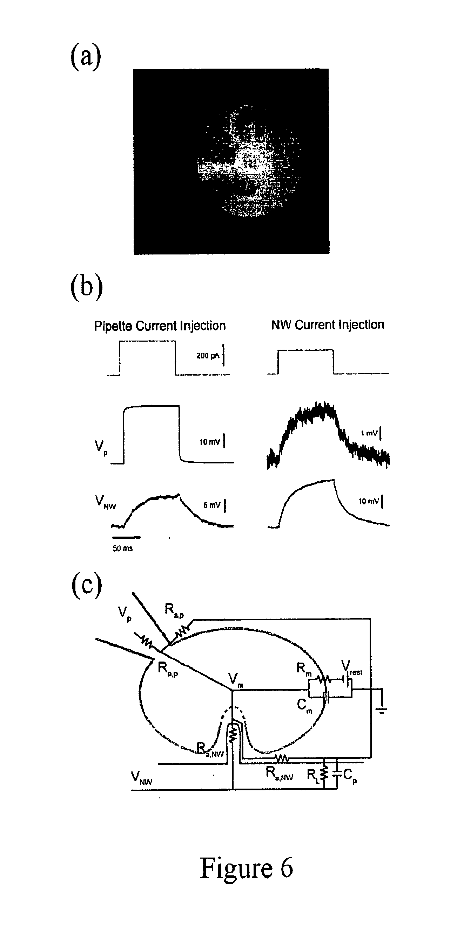

Figure 6(a) is a composite bright field and fluorescence image of a patched HEK293 cell on a vertical nanowire electrode array (VNEA) pad (0 DIV). Calcein was added to an intracellular recording solution to enable fluorescence imaging of the patched cell. Figure 6(b) shows voltage responses due to current injections that were recorded simultaneously using a patch pipette (Vp) and a VNEA pad (V_{NW}). Figure 6(c) shows an equivalent circuit model of a VNEA-cell interface. R_{a,NW} and R_{s,NW} represent the access and seal resistances of the NWs, respectively. R_{a,p} and R_{s,p} represent the access and seal resistances of the pipette, respectively. The equivalent circuit also includes the leak resistance due to uncoupled NWs or defects in electrode insulation (R_{L}) and the parasitic capacitance due to the device and associated electronics (Cₚ). The cell itself has a characteristic membrane resistance (Rₘ), capacitance (Cₘ), and resting potential (Vᵣₑₛₜ), all of which combine to determine the potential across the membrane (Vₘ) of the patched HEK293 cell.

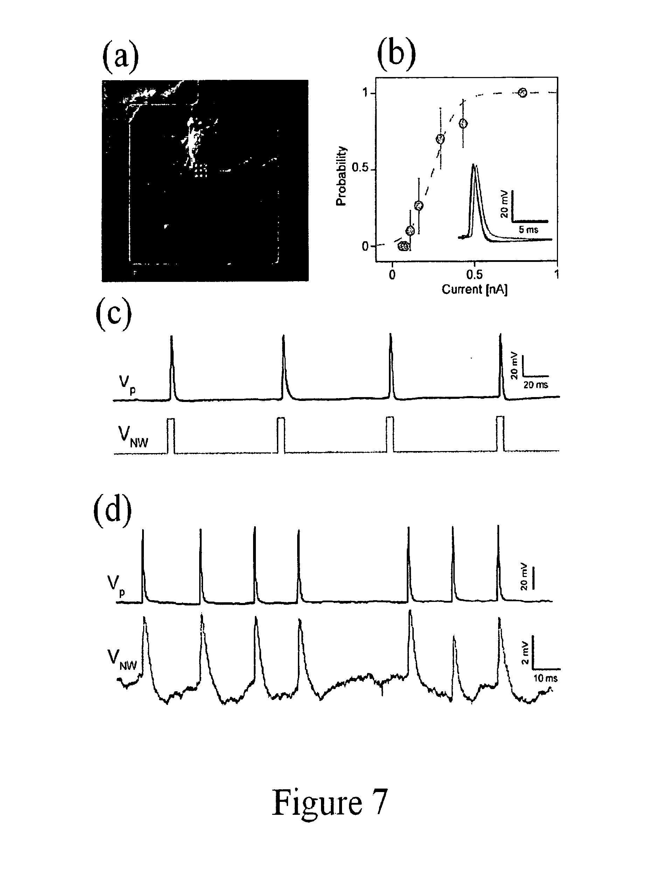

Figure 7(a) is a representative differential interference contrast microscopy image of a cortical neuron cultured on a VNEA pad (6 DIV). Figure 7(b) shows in dashed line the probability of action potential (AP) excitation plotted as a function of current injected by NWs, the plot having a sigmoidal shape. The probability is an average of 20 trials. Error bars represent 95% confidence intervals. Inset includes 5 consecutive time-aligned APs showing less than 1-ms jitter. Figure 7(c) includes APs stimulated by voltage pulses at a VNEA (bottom) and recorded using a patch pipette (top). Figure 7(d) includes APs stimulated using a patch pipette simultaneously recorded using both a VNEA pad (bottom) and a patch pipette (top).

### DETAILED DESCRIPTION

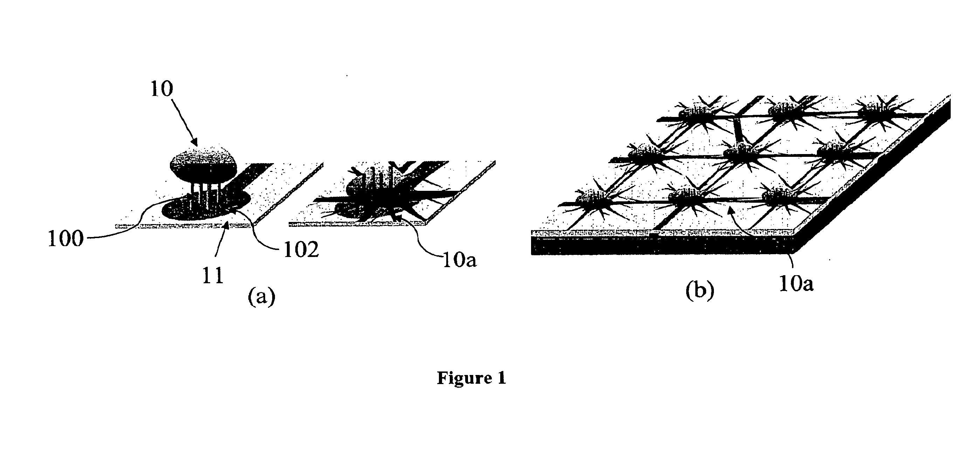

This description relates to a NW-based electrical device and a method of using the device to perform electrophysiological measurements. In one embodiment of using the device of this invention, as illustrated in Figure 1(a), neuron cell 10 (its axons or dendrites being labeled 10a) is brought into contact with substrate 11 having an array of vertical NWs 100 directly in contact with conductive track 102. Each NW, except its tip and the end contacting track 102, is covered with an insulating layer (not shown). The NWs 100 are in electrical communication with each other via track 102, which is electrically connected to an instrument (also not shown) for generating or detecting an electrical signal. In a preferred embodiment, the device of this invention has multiple electrically addressable tracks 102, each track electrically insulated from the other, as illustrated in Figure 1(b). The device can thus be used to study neuron-neuron interactions by, e.g., sending an electrical signal into one neuron in a neural network via the NWs inserted into this neuron and then detecting electrical signals from another neuron in the network via the NWs inserted into that neuron.

NWs used in this invention can be formed of any electrically conductive material, preferably inorganic material, such as silicon, metal, compound semiconductor, conductive oxide, and silicide. The insulating layer coated over the NWs is formed of material with low cytoxicity, such as silicon oxide, aluminum oxide, and silicon nitride. The tips of the NWs are either exposed (i.e., free of an insulating coating layer) or coated with an electrically conducting layer. The electrical conducting layer is also formed of material with low cytoxicity such as gold, silver, and platinum.

Two approaches are widely used for obtaining an array of NWs on a substrate. One is the so-called bottom-up approach, which essentially involves growing NWs from a precursor material. Taking chemical vapor deposition (CVD) for example, the NW growth process begins by placing or patterning catalyst or seed particles (usually with a diameter of 1 nm to a few hundred nanometers) atop a substrate; next, a precursor material is added to the catalyst or seed particles; and when the particles become saturated with the precursor, NWs begin to grow in a shape that minimizes the system's energy. By varying the precursor, substrate, catalyst/seed particles (e.g., size, density and deposition method on the substrate), and growth conditions, NWs can be made in a variety of materials, sizes, and shapes, at sites of choice. Another approach, the top-down process, essentially involves removing (e.g., by etching) predefined structures from a supporting substrate. For instance, the sites where the NWs are to be formed are first patterned into a soft mask (e.g., photoresist), which is either used to protect the sites that NWs will be formed during a subsequent etch or to pattern a hard mask; an etching step is subsequently performed (either wet or dry) to develop the patterned sites into three-dimensional wires.

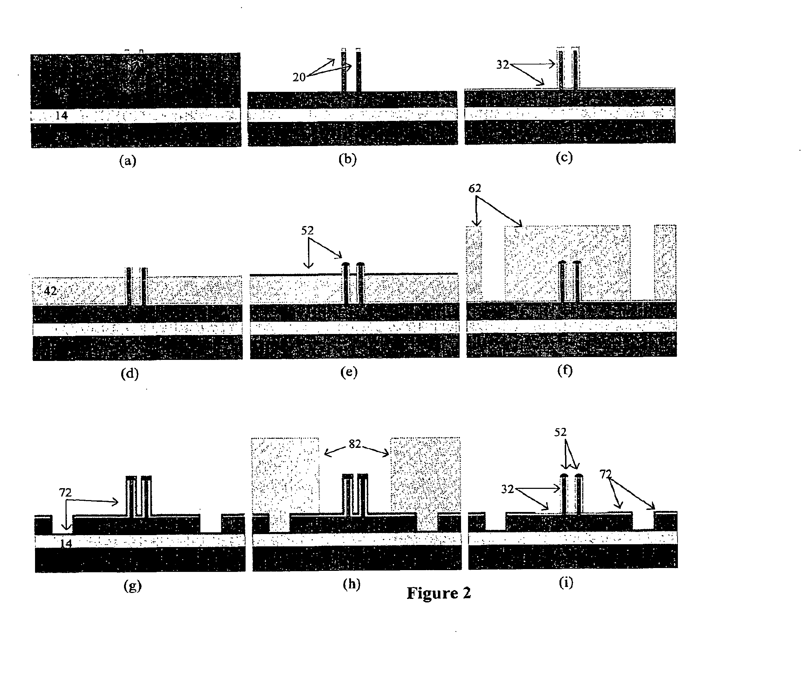

In one embodiment, the device of this invention is fabricated in the manner illustrated in Figure 2. First, in step (a), a pattern of disconnected silicon oxide dots 18 is created on top of a silicon wafer having a top silicon layer 12, an intermediate insulating layer 14 (e.g., silicon oxide), and a bottom silicon layer 16. The pattern of silicon oxide dots 18 then acts as a mask for etching the wafer to form vertical NWs 20 as shown in step (b). The top layer 12 is not completely removed in this step. Next, in step (c), the NWs and the remaining top layer 12 are coated with an insulating oxide layer 32 (e.g., SiO₂ and Al₂O₃) via deposition or thermal growth. Then in step (d), the oxide layer 32 and dots 18 on the tips of the NWs are stripped (e.g., using Hydrofluoric acid or reactive ion etching) after covering the wafer with a photoresist 42 with a thickness smaller than the length of the NWs. Afterwards, a metal layer 52 is deposited (e.g., via sputter coating, or thermal or electron-beam evaporation) as shown in step (e). The photoresist 42 is then removed, leaving behind metal 52 only at the tips of NWs. The NW electrodes are then made individually addressable by pattering electrode tracks using photolithography and etching through the Si device layer as illustrated in Figure 2, steps (f) through (i). More specifically, in step (f), photoresist 62 is patterned to define independent electrical access to each set of NWs; then in step (g), the silicon between electrode tracks is removed down to the insulating layer 14 (e.g., via reactive ion etching), and a conformal insulating layer 72 is deposited (e.g., Al₂O₃ via Atomic Layer Deposition); next, photoresist 82 is patterned to expose small windows around the NWs as shown in step (h); and lastly in step (i), layer 72 is selectively removed (e.g., by a chemically-selective wet or dry etch) leaving the NW metal tips 52 exposed, the regions around the NWs protected by a thin insulating layer 32, and the remainder of the device protected with an additional thick layer of insulation 72.

Device sensitivity to intracellular electrical signal can be manipulated by varying the NW density. Without wishing to be bound by the theory, an increase in the density of NWs on an electrically conductive track leads to an increase in the number of NWs penetrating a cell, which in turn improves the device sensitivity to electrical signals from the cell. Device sensitivity can also be manipulated by the size of the NWs. For example, NWs with a length comparable to the height of a cell can detect transmembrane signals with high sensitivity. On the other hand, NWs with a length comparable to the radius of a cell can detect more readily signals other than transmembrane ones.

Electrical voltage or current can be applied individually to each conductive track on which one ore more NWs are disposed to perform cell-specific measurement and stimulation either in a customized fashion or using standard protocols. As such, voltage clamp, current clamp, cyclic voltammetry measurements can be performed simultaneously on one device.

Other contemplated uses of the device described above include:

High-throughput screening for therapeutic effect and toxicology: Pharmaceutical compounds can be rapidly screened for their intended and unintended effects on various ion channels.

Diagnosis of channelopathies: Patient samples can be quickly and inexpensively tested for defects in ion channel behavior related to channelopathies.

Quantitative cellular and developmental biology: High-throughput electrophysiology assays can be performed on cells under various conditions to discover factors that relate to cellular electrophysiological behavior and development.

Intracellular electrochemical sensing and control: Through well-characterized electrochemical reactions, the device can be used to detect and control the intracellular electrochemical environment.

Cell-specific time-resolved controllable delivery of biomolecules: Voltage pulses can be controlled in time and space to perform specific delivery of genetic or pharmacological agents via electroporation, electrophoresis, or iontophoresis.

Neuronal electrophysiology: Measurements of neuronal electrophysiology, as well as synaptic plasticity and development can facilitate diagnosis and treatment of neuropsychiatric diseases.

High-precision long-term neural prosthetics: Intracellular electrical interfaces for monitoring and manipulating neuronal activity would provide a platform to treat neurological disorders and replace/bypass damaged functionality.

Brain machine interfaces: Intracellular electrodes could provide a neuronal interface for the control and feedback of prosthetic limbs and other external devices.

The following examples are provided for illustrative purposes

### Example 1: Preparation of Si NW arrays by etching

An array of Si NWs on a silicon substrate was formed via several lithography, etching, and deposition steps.

First, an etch mask was defined via electron beam lithography (EBL). The silicon on insulator wafer was coated with XR-1541 6% solids negative E-beam resist (Dow Corning) at 2000 RPM to produce a layer of resist approximately 200 nm thick. The wafer was then baked for 2 minutes at 225 °C before electron beam exposure. The Raith-150 EBL tool was used to define 100 nm diameter circles at the locations desired for NW formation. After exposure at a dose of 1000 µC/cm² the wafer was baked again at 225 °C for 4 minutes. The pattern was then developed for 15 seconds in 25% Tetramethylammonium hydroxide (TMAH). The resist left behind after developing acted as a hard mask for the subsequent etch process. An inductively-coupled plasma (ICP) of HBr:O₂ was applied for 10 minutes in an ICP-RIE system (SURFACE TECHNOLOGY SYSTEMS) to afford an array of Si NWs (average length: 1000 nm; average diameter: 150 nm; density: 0.5 wire/µm²). The resist mask was then removed by dipping the wafer in 49% hydrofluoric acid. The NWs were then insulated using low pressure chemical vapor deposition (LPCVD) of SiO₂ at 800 °C. To remove the SiO₂ at the NW tips, S1818 photoresist (Microchem) was spun at 3000 RPM and then stripped back using an O₂ plasma (Unaxis RIE) to leave a 500 nm film on the Si substrate. The tips of the NWs which protrude above this layer were then etched (STS ICP-RIE) using a CF₄ plasma to remove the SiO₂ covering the tip. The device was then treated with a 1-min O₂ plasma descum followed by a 10-second dip in buffered oxide etch (BOE) 7:1. The substrate was then loaded into a thermal evaporator where 10 nm Ni adhesion layer was evaporated followed by a 70 nm layer of Au. The resist was then dissolved for several hours using Remover PG (MicroChem) at 80 °C leaving the metal layer only at the NW tips. Electrode tracts were then patterned by spinning S1818 photoresist (Microchem) on the wafer at 3000 PRM. After baking the wafer for 1 minute at 115 °C, UV contact lithography was used to expose the regions between electrodes. The exposed resist was then developed away using MF-319 (Microchem). The remaining resist served as a mask for ICP-RIE etching (STS) of the Si substrate using a C₄F₈:SF₆ plasma. After stripping the resist with Remover PG, the substrate was coated with 100 nm of Al₂O₃ using atomic layer deposition (ALD) (Cambridge NanoTech). Using contact lithography, 20-micron diameter areas were exposed around the NWs, as well as 1 x 0.5 mm areas for contact pads. After development, the Al₂O₃ in these regions was removed using TransEtch (Transene). The photoresist was removed and reapplied and the contact regions alone were exposed and developed. After stripping the SiO₂ in these regions using BOE 7:1, a Ni/Au layer was evaporated as before and the photoresist was stripped.

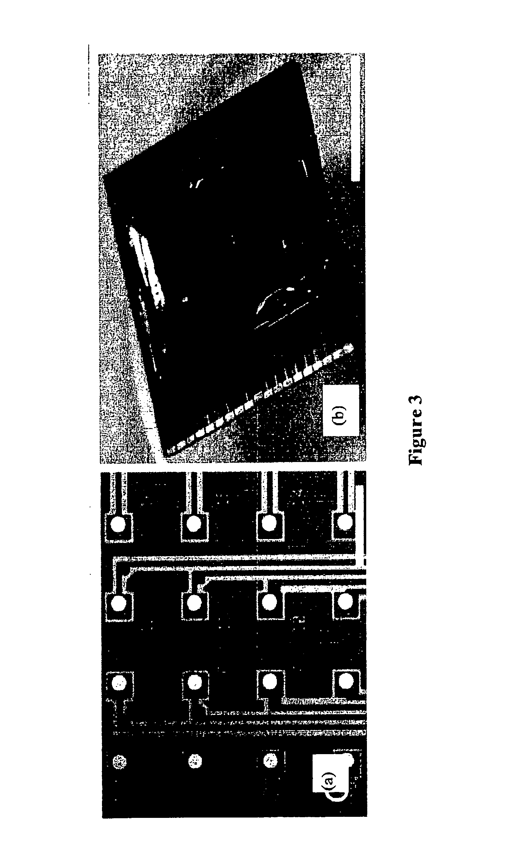

As shown in Figure 3(a), a device thusly produced contains 16 Si tracks each having an array of Si NWs with metal tips disposed thereon. Each individual set of Si NWs is electrically addressable via their own Si track connected to an Au contact pad that resides outside the cell culture solution. See Figure 3(b).

### Example 2: Plating neuron cells on a NW array

Prior to plating rat hippocampal neurons, the substrates were cleaned in soap and water, sterilized with ethanol, and left in poly-l-lysine for 20 minutes before being rinsed with sterile-filtered DD H₂O. Dissociated E18 embryonic hippocampal neurons were then plated on Si NW substrates as small drops so as to yield a final density of ∼5 × 10⁴ cells/cm². After incubation for 15 min, Neurobasal/B27 media was added. A 50% media swap was performed on the fourth, seventh, and eleventh days.

Prior to imaging, the cells were fixed in a solution of 4% glutaraldehyde in 0.1 M sodium cacodylate (2 h), rinsed, and fixed again in a 1% solution of osmium tetroxide in 0.1 M sodium cacodylate (2 h). The samples were then dehydrated in gradually increasing concentrations of ethanol (from 50-100%) in water, dried in a critical point dryer, and sputter-coated with a few nanometers of platinum/palladium.

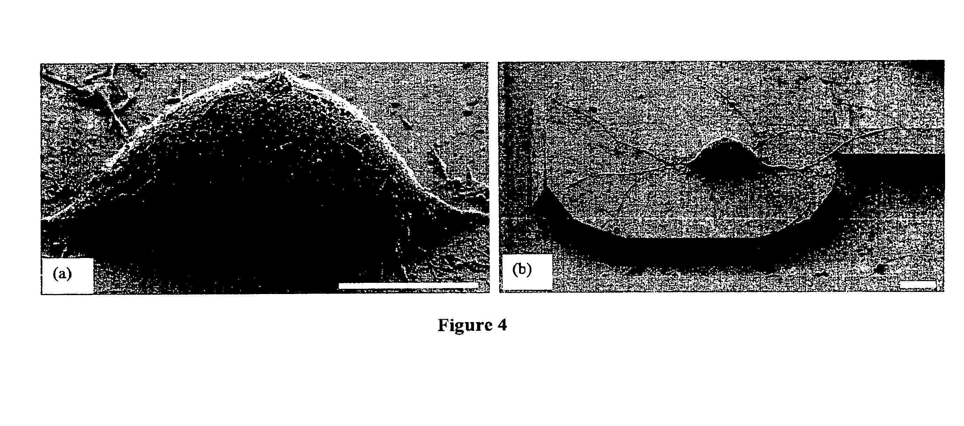

As demonstrated by the SEM image of Figure 4(a), the cell was penetrated by a number of NWs of the array. Further, as shown in Figure 4(b), the NWs penetrating the cell were disposed on one Si track and therefore were insulated from NWs that were disposed on other Si tracks.

### Example 3: Electrical measurements

HEK cells were plated on top of the device produced in Example 1 in a manner similar to that described in Example 2 for patch clamp measurements and current injection via the Au-tipped Si NWs.

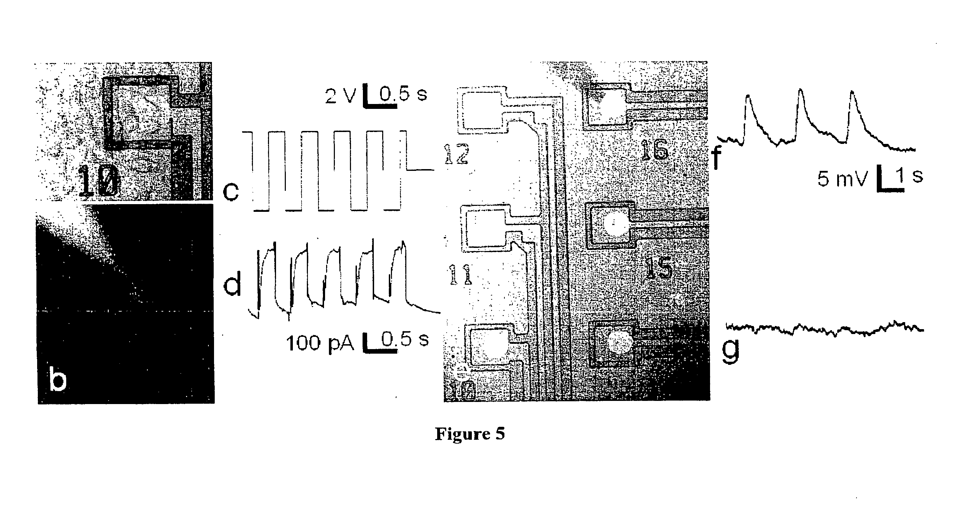

As shown in Figure 5(a), i.e., a bright field image of the cell-plated device, a few HEK cells were seeded on a Si track number 10 of the device prior to patch clamp measurements and current injection via the Au-tipped Si NWs. Then a patch pipette back-filled with fluorescein diacetate (FDA) was brought into contact with one of the HEK cells. As shown in Figure 5(b), an overlay of a bright field image of a Si track number 10 (shown as the dark background in the figure) and a fluorescence image showing the patch pipette (shown as a bright cone), the measured HEK cell (shown as a bright dot at the tip of the bright cone) also fluoresced due to FDA diffusion from the patch pipette. Next, a negative bias in the shape shown in Figure 5(c) was applied to the Au-tipped Si NWs on Si track number 10 and the cell response was measured by whole cell voltage clamp recording using the patch pipette. As shown in Figure 5(d), negative currents were successfully injected into the tested cell via the Au-tipped Si NWs.

The electrically-addressable NW device was also used to measure changes in the Nernst potential. As shown in Figure 5(e), a bright field image, a patch pipette was positioned above Si track number 16. The pipette was back-filled with a pH 7.0 buffer, which was periodically injected into the pH 7.2 cell culture bath. Operating in current clamp mode, the current flowing through the Si NWs was clamped to -0.5 nA. By recording the voltage required to maintain this -0.5 nA current, the change in the Nernst potential was measured via the Si NWs. As calculated, the pH difference of 0.2 would generate a Nernst potential of 12 mV.

Figures 5(f) and 5(g) respectively show the results from the current clamp measurements using the Si NWs at track numbers 16 and 14, which was 400 µm from the patch pipette above track number 16. As indicated by the current clamp results shown in Figure 5(f), the device was sensitive to potential changes less than 10 mV. Further, the constant voltage measured from track number 14 as shown in Figure 5(g) verified that the Si NWs in track number 16 were active in obtaining the current clamp data as shown in Figure 5(f).

### Example 4: Characterization of the VNEA-cell electrical interface

Operation protocols for a vertical nanowire electrode array (VNEA) device were developed and optimized using HEK293 cells as a model system.

HEK293 cells are advantageous for establishing stimulation and recording procedures as they require a short culture time before electrical interrogation (only a few hours) and their membrane resistance remains constant within 15 mV of the resting membrane potential as described in Thomas, et al., J. Pharmacol. Toxicol. Methods, 51, 187 (2005).

To determine the device parameters that characterize the VNEA-cell interface, an experiment was performed using whole-cell patch-clamp recordings on HEK293 cells residing directly on top of the NWs. Voltages and currents were monitored simultaneously using both a patch pipette and a VNEA pad. See Figure 6(a). In more than half of the cases, the membrane potential changed immediately upon VNEA current injection, suggesting that the NWs had spontaneously penetrated the cell's membrane. If the membrane potential of a cell did not change significantly upon current injection (i.e. when the NWs were not inside the cell), to promote NW penetration, the cell membrane was permeabilized using a short voltage pulse (∼ -3V, 100 ms duration) as described in Rols, et al., Biophys. J. 58, 1089 (1990).

Once the NWs had access to the cell's interior, the VNEA device was used to measure and control the membrane potential of the cell by leveraging electrochemistry at the NW tips. Specifically, it was found that when current was injected through the patch pipette, the voltage measured at the NWs (V_{NW}), which is required to maintain a fixed current through the NW channel, changed (see Figure 6(b), left panel), demonstrating that the NWs can be used to monitor the membrane potential in a current clamp mode. Similarly, when current was injected through the NWs, the voltage, measured at the patch pipette (Vp), changed (see Figure 6(b), right panel), indicating that NW-based control over membrane potential is also possible. It should be noted that when current was injected through the pipette, voltage changes measured at the NWs were ∼3 times smaller than those measured at the pipette. Conversely, when current was injected through the NWs, voltage changes measured at the pipette where ∼10 times smaller than those measured at the NWs. Analysis of data, such as those shown in Figure. 6(b), using the equivalent circuit model depicted in Figure 6(c), enabled the determination of all of the parameters that specify the electrical coupling between the cell and the NWs.

In particular, this analysis shows that the seal resistance (R_{s,NW}) between the NWs and the cell membrane ranged between 100 and 500 MΩ, and that the access resistance of the NW electrodes (R_{a,NW}) (which includes the intrinsic NW resistance and the resistance at the electrochemical junction) was -300 MΩ under typical operating conditions (V_{NW} ∼ -1.5 V). Moreover, the parasitic capacitance (Cₚ) of a typical VNEA pad and its associated electronics was determined to be ∼150 pF, resulting in an RC time constant on the order of 10 ms. Although this RC component filtered the voltage waveform measured at the NWs (as compared to that measured at a patch pipette), this distortion could be easily corrected using a deconvolution procedure.

### Example 5: Stimulation and recording of rat cortical neurons using a VNEA

Once the device characterization and protocol optimization were complete, the VNEA was used to perform high-fidelity intracellular stimulation and recording of rat cortical neurons (see Figure 7(a)).

Typically, these neurons were interrogated after 6 to 14 divisions (DIV) to allow electrophysiological development and the formation of synaptic connections.

As shown in Figure 7(c), current pulses injected into neurons via the NWs reliably evoked neuronal action potentials (APs) as recorded via simultaneous whole-cell patch clamp. The stimulation probability followed a sigmoidal dependence on the magnitude of the NW-injected current (see Figure 7(b)), similar to that reported for patch pipette stimulation (Kole, et al., Nat. Neurosci. 2008, 11, 1253). Moreover, the VNEA, operating in current-clamp mode, could be used to monitor individual APs evoked by the patch pipette. For single-shot AP measurements, the signal-to-noise ratio was typically 100 or greater (see Figure 7(d)). An average of five waveforms obtained under identical experimental conditions could achieve a signal-to-noise ratio exceeding 1000.

The invention is as defined in the appended claims that follow.

## Claims

1. An electrical device comprising:
a substrate having a surface, and
a plurality of electrically conductive nanowires, each of which has a first end and a second end,
wherein the substrate and each of the nanowires are coated with an electrically insulating layer except for the first and second ends of each of the nanowires, the first end is attached to the surface, and the second end is coated with an electrically conductive layer.

2. The device of claim 1, wherein each of the nanowires is formed of a semiconductor, a compound semiconductor, a metal oxide, a metal, carbon, boron nitride, or a combination thereof, preferably wherein the semiconductor is silicon.

3. The device of claim 1, wherein the electrically insulating layer is formed of an inorganic material, preferably wherein the inorganic material is an oxide or a nitride, preferably wherein the inorganic material is silica, alumina, hafnium oxide, or silicon nitride, or wherein the electrically insulating layer is formed of an organic material, preferably wherein the organic material is a photoresist or an electron beam resist, preferably wherein the organic material is Parylene, polydimethylsiloxane, methyl methacrylate, polymethylmethacrylate, or SU-8 photoresist.

4. The device of claim 1, wherein the electrically conductive layer is formed of a semiconductor, a compound semiconductor, a metal, a metal oxide, a metal nitride, or a combination thereof.

5. The device of claim 4, wherein the electrically conductive layer is formed of a metal, preferably wherein the metal is Ag, Au, Pt, Ni, Al, Pd, W, Ti, or Cr.

6. The device of claim 4, wherein the electrically conductive layer is formed of a metal oxide or a metal nitride, preferably wherein the electrically conductive layer is formed of indium tin oxide or titanium nitride.

7. The device of claim 4, wherein the electrically conductive layer is formed of a metal silicide, preferably wherein the metal silicide is PtSi, Pt₂Si, NiSi, Ni₂Si, NiSi₂, WSi₂, Pd₂Si, TiSi₂, or CrSi₂.

8. The device of claim 4, wherein the electrically conductive layer is formed of a semiconductor, preferably wherein the semiconductor is formed of silicon.

9. The device of claim 1, wherein the electrically conductive layer includes a metal top layer and a metal silicide intermediate layer between the metal top layer and the second end, preferably wherein the metal silicide intermediate layer is a silicide of Pt, Ni, W, Pd, Ti, Cr, Yb, Er, Tb, Dy, Gd, Ho, Y, Hf, Zr, Ta, Co, V, Mo, Rh, Ir, or a combination thereof, preferably wherein the silicide is PtSi, Pt₂Si, NiSi, Ni₂Si, NiSi₂, WSi₂, Pd₂Si, TiSi₂, CrSi2, YbSi₂, ErSi₂, TbSi₂, DySi₂, GdSi₂, HoSi₂, YSi₂, HfSi, ZrSi₂, TaSi₂, CoSi₂, VSi₂, CoSi, MoSi₂, RhSi, Ir₂Si₃, IrSi, or IrSi₃.

10. The device of claim 1, wherein the device has a density of the first plurality of nano wires per unit area between 0.05 and 10 wires µm⁻², between 0.5 and 5 wires µm⁻² or between 1 and 2 wires µm⁻².

11. The device of claim 1, wherein the plurality of nanowires are attached to the surface along a substantially vertical direction to the surface.

12. The device of claim 1, wherein at least two of the plurality of nanowires are in electrical communication with each other or at least two of the plurality of nanowires are electrically insulated from each other.

13. The device of claim 1, wherein the plurality of electrically conductive nanowires includes a first plurality of electrically conductive nanowires and a second plurality of electrically conductive nanowires, the first plurality of nanowires are electrically insulated from the second plurality of nanowires, the first plurality of nanowires are in electrical communication with each other, and the second plurality of nanowires are in electrical communication with each other.

14. A method of sending or receiving an electrical signal to or from a biological cell in vitro the method comprising:
providing a device of claim 1, and
contacting a first biological cell cultured on a surface with the device to allow penetration of a first nanowire into the first cell, whereby one end of the first nanowire is located inside the first cell for sending or receiving the electrical signal.

15. The method of claim 14, further comprising contacting a second biological cell with the device to allow penetration thereinto of a second nanowire that is electrically insulated from the first nanowire, whereby one end of the second nanowire is located inside the second cell for sending and receiving a second electrical signal, preferably wherein each of the first and second cells is independently a neuron, a neuroblast, an HEK cell, a HeLa cell, an oocyte, a beta cell, a myocyte, an osteocyte, a fibroblast, a macrophage, or an induced pluripotent stem cell, preferably wherein both the first and second cells are neurons.

## Patentansprüche

1. Elektrische Vorrichtung, umfassend:
ein Substrat mit einer Oberfläche und
mehrere elektrisch leitfähige Nanodrähte, von denen jeder ein erstes Ende und ein zweites Ende hat,
wobei das Substrat und jeder der Nanodrähte mit einer elektrisch isolierenden Schicht beschichtet sind, mit Ausnahme der ersten und zweiten Enden von jedem der Nanodrähte, wobei das erste Ende an der Oberfläche befestigt ist, und das zweite Ende mit einer elektrisch leitfähigen Schicht beschichtet ist.

2. Vorrichtung nach Anspruch 1, in der jeder der Nanodrähte aus einem Halbleiter, einem Verbindungshalbleiter, einem Metalloxid, einem Metall, Kohlenstoff, Bornitrid oder einer Kombination davon gebildet ist, wobei der Halbleiter vorzugsweise Silicium ist.

3. Vorrichtung nach Anspruch 1, in der die elektrisch isolierende Schicht aus einem anorganischen Material gebildet ist, wobei das anorganische Material vorzugsweise ein Oxid oder ein Nitrid ist, wobei das anorganische Material vorzugsweise Silica, Alumina, Hafniumoxid, oder Siliciumnitrid ist, oder in der die elektrisch isolierende Schicht aus einem organischen Material gebildet ist, wobei das organische Material vorzugsweise ein Photoresist oder ein Elektronenstrahl-Resist ist, wobei das organische Material vorzugsweise Parylene, Polydimethylsiloxan, Methylmethacrylat, Polymethylmethacrylat oder SU-8 Photoresist ist.

4. Vorrichtung nach Anspruch 1, in der die elektrisch leitfähige Schicht aus einem Halbleiter, einem Verbindungshalbleiter, einem Metall, einem Metalloxid, einem Metallnitrid oder einer Kombination davon gebildet ist.

5. Vorrichtung nach Anspruch 4, in der die elektrisch leitfähige Schicht aus einem Metall gebildet ist, wobei das Metall vorzugsweise Ag, Au, Pt, Ni, Al, Pd, W, Ti oder Cr ist.

6. Vorrichtung nach Anspruch 4, in der die elektrisch leitfähige Schicht aus einem Metalloxid oder einem Metallnitrid gebildet ist, wobei die elektrisch leitfähige Schicht vorzugsweise aus Indium-Zinn-Oxid oder Titannitrid gebildet ist.

7. Vorrichtung nach Anspruch 4, in der die elektrisch leitfähige Schicht aus einem Metallsilicid gebildet ist, wobei das Metallsilicid vorzugsweise PtSi, Pt₂Si, NiSi, Ni₂Si, NiSi₂, WSi₂, Pd₂Si, TiSi₂ oder CrSi₂ ist.

8. Vorrichtung nach Anspruch 4, in der die elektrisch leitfähige Schicht aus einem Halbleiter gebildet ist, wobei der Halbleiter vorzugsweise aus Silicium gebildet ist.

9. Vorrichtung nach Anspruch 1, in der die elektrisch leitfähige Schicht eine Metall-Deckschicht und eine Metallsilicid-Zwischenschicht umfasst, die zwischen der Metall-Deckschicht und dem zweiten Ende angeordnet ist, wobei die Metallsilicid-Zwischenschicht vorzugsweise ein Silicid von Pt, Ni, W , Pd, Ti, Cr, Yb, Er, Tb, Dy, Gd, Ho, Y, Hf, Zr, Ta, Co, V, Mo, Rh, Ir, oder einer Kombination davon ist, wobei das Silicid vorzugsweise PtSi, Pt₂Si, NiSi, Ni₂Si, NiSi₂, WSi₂, Pd₂Si, TiSi₂, CrSi₂, YbSi₂, ErSi₂, TbSi₂, DySi₂, GdSi₂, HoSi₂, YSi₂, HfSi, ZrSi₂, TaSi₂, CoSi₂, VSi₂, CoSi, MoSi₂, RhSi, Ir₂Si₃, IrSi, or IrSi₃ ist.

10. Vorrichtung nach Anspruch 1, wobei die Vorrichtung eine Dichte der ersten mehreren Nanodrähte pro Flächeneinheit zwischen 0,05 und 10 Drähte µm⁻², zwischen 0,5 und 5 Drähte µm⁻² oder zwischen 1 und 2 Drähte µm⁻² hat.

11. Vorrichtung nach Anspruch 1, in der die mehreren Nanodrähte an der Oberfläche entlang einer im Wesentlichen vertikalen Richtung zu der Oberfläche befestigt sind.

12. Vorrichtung nach Anspruch 1, in der zumindest zwei der mehreren Nanodrähte miteinander elektrisch verbunden sind oder zumindest zwei der mehreren Nanodrähte voneinander elektrisch isoliert sind.

13. Vorrichtung nach Anspruch 1, in der die mehreren elektrisch leitfähigen Nanodrähte erste mehrere elektrisch leitfähige Nanodrähte und zweite mehrere elektrisch leitfähige Nanodrähte umfassen, wobei die ersten mehreren Nanodrähte von den zweiten mehreren Nanodrähten elektrisch isoliert sind, die ersten mehreren Nanodrähte miteinander elektrisch verbunden sind, und die zweiten mehreren Nanodrähte miteinander elektrisch verbunden sind.

14. Verfahren zum Senden oder Empfangen eines elektrischen Signals zu oder von einer biologischen Zelle *in vivo,* wobei das Verfahren umfasst:
Bereitstellen einer Vorrichtung nach Anspruch 1, und In-Kontakt-Bringen einer ersten biologischen Zelle, die auf einer Oberfläche kultiviert wird, mit der Vorrichtung, um das Eindringen eines ersten Nanodrahts in die erste Zelle zu ermöglichen, wobei ein Ende des ersten Nanodrahts in der ersten Zelle zum Senden oder Empfangen des elektrischen Signals platziert wird.

15. Verfahren nach Anspruch 14, ferner umfassend:
In-Kontakt-Bringen einer zweiten biologischen Zelle mit der Vorrichtung, um das Eindringen eines zweiten Nanodrahts, der von dem ersten Nanodraht elektrisch isoliert ist, in die zweite Zelle zu ermöglichen, wobei ein Ende des zweiten Nanodrahts in der zweiten Zelle zum Senden oder Empfangen des elektrischen Signals platziert wird, wobei vorzugsweise jede der ersten und zweiten Zellen unabhängig voneinander ein Neuron, ein Neuroblast, eine HEK-Zelle, eine HeLa-Zelle, eine Eizelle, eine Beta-Zelle, ein Myozyt, ein Osteozyt, ein Fibroblast, ein Makrophage oder eine induziert pluripotente Stammzelle ist, wobei vorzugsweise sowohl die erste als auch die zweite Zellen Neuronen sind.

## Revendications

1. Dispositif électrique comprenant :
un substrat ayant une surface, et
une pluralité de nanofils électro-conducteurs, dont chacun a une première extrémité et une deuxième extrémité,
dans lequel le substrat et chacun des nanofils sont revêtus d'une couche d'isolation électrique à l'exception des première et deuxième extrémités de chacun des nanofils, la première extrémité est fixée à la surface, et la deuxième extrémité est revêtue d'une couche électro-conductrice.

2. Dispositif selon la revendication 1, dans lequel chacun des nanofils est formé d'un semi-conducteur, d'un semi-conducteur composé, d'un oxyde de métal, d'un métal, de carbone, d'un nitrure de bore, ou d'une combinaison de ceux-ci, préférablement dans lequel le semi-conducteur est le silicium.

3. Dispositif selon la revendication 1, dans lequel la couche d'isolation électrique est formée d'un matériau inorganique, préférablement dans lequel le matériau inorganique est un oxyde ou un nitrure, préférablement dans lequel le matériau inorganique est une silice, une alumine, un oxyde d'hafnium, ou un nitrure de silicium, ou dans lequel la couche d'isolation électrique est formée d'un matériau organique, préférablement dans lequel le matériau organique est une résine photosensible ou une résine sensible à un faisceau d'électrons, préférablement dans lequel le matériau organique est un parylène, un polydiméthylsiloxane, un méthacrylate de méthyle, un poly(méthacrylate de méthyle), ou une résine photosensible SU-8.

4. Dispositif selon la revendication 1, dans lequel la couche électro-conductrice est formée d'un semi-conducteur, d'un semi-conducteur composé, d'un métal, d'un oxyde de métal, d'un nitrure de métal, ou d'une combinaison de ceux-ci.

5. Dispositif selon la revendication 4, dans lequel la couche électro-conductrice est formée d'un métal, préférablement dans lequel le métal est Ag, Au, Pt, Ni, Al, Pd, W, Ti, ou Cr.

6. Dispositif selon la revendication 4, dans lequel la couche électro-conductrice est formée d'un oxyde de métal ou d'un nitrure de métal, préférablement dans lequel la couche électro-conductrice est formée d'un oxyde d'indium-étain ou d'un nitrure de titane.

7. Dispositif selon la revendication 4, dans lequel la couche électro-conductrice est formée d'un siliciure de métal, préférablement dans lequel le siliciure de métal est PtSi, Pt₂Si, NiSi, Ni₂Si, NiSi₂, WSi₂, Pd₂Si, TiSi₂, ou CrSi₂.

8. Dispositif selon la revendication 4, dans lequel la couche électro-conductrice est formée d'un semi-conducteur, préférablement dans lequel le semi-conducteur est formé de silicium.

9. Dispositif selon la revendication 1, dans lequel la couche électro-conductrice inclut une couche supérieure de métal et une couche intermédiaire de siliciure de métal entre la couche supérieure de métal et la deuxième extrémité, préférablement dans lequel la couche intermédiaire de siliciure de métal est un siliciure de Pt, Ni, W, Pd, Ti, Cr, Yb, Er, Tb, Dy, Gd, Ho, Y, Hf, Zr, Ta, Co, V, Mo, Rh, Ir, ou une combinaison de ceux-ci, préférablement dans lequel le siliciure est PtSi, Pt₂Si, NiSi, Ni₂Si, NiSi₂, WSi₂, Pd₂Si, TiSi₂, CrSi₂, YbSi₂, ErSi₂, TbSi₂, DySi₂, GdSi₂, HoSi₂, YSi₂, HfSi, ZrSi₂, TaSi₂, CoSi₂, VSi₂, CoSi, MoSi₂, RhSi, Ir₂Si₃, IrSi, ou IrSi₃.

10. Dispositif selon la revendication 1, dans lequel le dispositif a une densité de la première pluralité de nanofils par unité de superficie entre 0,05 et 10 fil(s) *µ*m⁻², entre 0,5 et 5 fil (s) *µ*m⁻² ou entre 1 et 2 fil (s) *µ*m⁻².

11. Dispositif selon la revendication 1, dans lequel la pluralité de nanofils sont fixés à la surface le long d'un sens sensiblement vertical par rapport à la surface.

12. Dispositif selon la revendication 1, dans lequel au moins deux parmi la pluralité de nanofils sont en communication électrique l'un avec l'autre, ou bien au moins deux parmi la pluralité de nanofils sont électriquement isolés l'un de l'autre.

13. Dispositif selon la revendication 1, dans lequel la pluralité de nanofils électro-conducteurs inclut une première pluralité de nanofils électro-conducteurs et une deuxième pluralité de nanofils électro-conducteurs, la première pluralité de nanofils sont électriquement isolés de la deuxième pluralité de nanofils, la première pluralité de nanofils sont en communication électrique les uns avec les autres, et la deuxième pluralité de nanofils sont en communication électrique les uns avec les autres.

14. Procédé pour envoyer ou recevoir un signal électrique à une ou d'une cellule biologique *in vitro,* le procédé comprenant :
la prévision d'un dispositif selon la revendication 1, et
la mise en contact d'une première cellule biologique en culture sur une surface avec le dispositif pour permettre une pénétration d'un premier nanofil dans la première cellule, d'où il résulte qu'une extrémité du premier nanofil est située à l'intérieur de la première cellule pour envoyer ou recevoir le signal électrique.

15. Procédé selon la revendication 14, comprenant en outre la mise en contact d'une deuxième cellule biologique avec le dispositif pour permettre une pénétration dans celle-ci d'un deuxième nanofil qui est électriquement isolé du premier nanofil, d'où il résulte qu'une extrémité du deuxième nanofil est située à l'intérieur de la deuxième cellule pour envoyer et recevoir un deuxième signal électrique, préférablement dans lequel chacune des première et deuxième cellules est indépendamment un neurone, un neuroblaste, une cellule HEK, une cellule HeLa, un oocyte, une cellule bêta, un myocyte, un ostéocyte, un fibroblaste, un macrophage, ou une cellule souche pluripotente induite, préférablement dans lequel les première et deuxième cellules sont toutes les deux des neurones.
